# EUROPEAN PATENT SPECIFICATION

(11) **EP 3 801 577 B1**
(45) Date of publication and mention of the grant of the patent: **19.02.2025**
(21) Application number: 19811896.0
(22) Date of filing: 30.05.2019
(51) Int. Cl.: A61K 35/39, A61P 3/10, C12N 5/071

(54) **METHODS AND COMPOSITIONS COMPRISING TANKYRASE INHIBITORS FOR GENERATING INSULIN PRODUCING CELLS**
VERFAHREN UND ZUSAMMENSETZUNGEN MIT TANKYRASE-INHIBITOR ZUR ERZEUGUNG VON INSULINPRODUZIERENDEN ZELLEN
PROCÉDÉS ET COMPOSITIONS COMPRENANT DES INHIBITEURS DE TANKYRASE POUR PRODUIRE DES CELLULES PRODUISANT DE L'INSULINE

(30) Priority: 31.05.2018 US 201862678498 P
(43) Date of publication of application: 14.04.2021
(73) Proprietor: University Health Network (UHN), Toronto, Ontario M5G 2C4 (CA)
(72) Inventor: NOSTRO, Maria, Cristina, Toronto, Ontario M5C 3H8 (CA); SARANGI, Farida, Thornhill, Ontario L3T 7C8 (CA); KORYTNIKOV, Roman, Thornhill, Ontario L4J 0A1 (CA); MCGAUGH, Emily, Brampton, Ontario L6Z 2W6 (CA); POON, Hoi Chun, Frankie, Toronto, Ontario M5S 0C5 (CA)
(74) Representative: Kolster Oy Ab
(86) International application number: PCT/CA2019/000079
(87) International publication number: WO 2019/227198

(56) References cited:
- EP-A1- 3 178 924
- WO-A1-2013/163739
- WO-A1-2016/170069
- WO-A1-2016/172564
- WO-A1-2017/149025
- WO-A1-2020/033879
- HUANG YONGJIAN ET AL: "A molecular mechanism for the generation of ligand-dependent differential outputs by the epidermal growth factorreceptor", 30 November 2021 (2021-11-30), XP055915555, Retrieved from the Internet <URL:https://elifesciences.org/articles/73218> [retrieved on 20220426]
- BODDY SARAH L ET AL: "Inner ear progenitor cells can be generated in vitro from human bone marrow mesenchymal stem cells", REGENERATIVE MEDICINE, FUTURE MEDICINE LTD, GB, vol. 7, no. 6, 1 November 2012 (2012-11-01), pages 757 - 767, XP009189899, ISSN: 1746-0751, DOI: 10.2217/RME.12.58
- CLAUDIA DAVENPORT ET AL: "Anterior-Posterior Patterning of Definitive Endoderm Generated from Human Embryonic Stem Cells Depends on the Differential Signaling of Retinoic Acid, Wnt-, and BMP-Signaling : A-P Patterning of the Definitive Endoderm", STEM CELLS, vol. 34, no. 11, 4 July 2016 (2016-07-04), pages 2635 - 2647, XP055657494, ISSN: 1066-5099, DOI: 10.1002/stem.2428
- NOSTRO, MC ET AL.: "Efficient Generation ofNKX6-1 + Pancreatic Progenitors from Multiple Human Pluripotent Stem Cell Lines", STEM CELL REPORTS, vol. 4, 14 April 2015 (2015-04-14), pages 591 - 604, XP055223322, ISSN: 2213-6711, DOI: 10.1016/j.stemcr.2015.02.017
- PAGLIUCA FW ET AL.: "Generation of Functional Human Pancreatic fi Cells In Vitro", CELL, vol. 159, 9 October 2014 (2014-10-09), pages 428 - 439, XP029073423, ISSN: 1097-4172
- REZANIA A ET AL.: "Reversal of diabetes with insulin-producing cells derived in vitro from human pluripotent stem cells", NATURE BIOTECHNOLOGY, vol. 32, no. 11, November 2014 (2014-11-01), pages 1121 - 1133, XP055200937, ISSN: 1097-4172, DOI: 10.1038/nbt.3033
- ZHANG H ET AL.: "Epidermal Growth Factor Promotes Proliferation and Migration of Follicular Outer Root Sheath Cells via Wnt/fi-Catenin Signaling", CELLULAR PHYSIOLOGY AND BIOCHEMISTRY, vol. 39, 29 June 2016 (2016-06-29), pages 360 - 370, XP055659569, ISSN: 1421-9778
- VETHE, H ET AL.: "The Effect of Wnt Pathway Modulators on Human iPSC-Derived Pancreatic Beta Cell Maturation", FRONTIERS IN ENDOCRINOLOGY, vol. 10, no. 293, 8 May 2019 (2019-05-08), pages 1 - 13, XP055659574, ISSN: 1664-2392

## Description

### Field

The disclosure relates to in vitro methods and in vitro uses of compositions for producing insulin-producing beta-like cells.

### Background

The generation of insulin-producing beta-like cells from human pluripotent stem cells ("hPSCs"; including human embryonic stem cells, "hESCs"; and human induced pluripotent stem cells, "hiPSCs") can provide a renewable source of human endocrine cells for several applications: 1) transplantation into patients as a future treatment option for diabetes; 2) developmental and disease modelling; and 3) drug discovery and testing for pancreas-associated diseases. In order for these applications to be realized, differentiation strategies need to be identified that are effective and consistent across different cell lines and laboratories. Here, we describe the use of specific tankyrase inhibitors to generate PDX1+/NKX6-1+ pancreatic progenitor cells with increased capability to generate insulin-expressing cells.

While there are several reports on methods for obtaining PDX1+/NKX6-1+ positive pancreatic progenitor cells using inter alia tankyrase inhibitors (EP3178924; Davenport et al., Stem Cells., 2016; 34(11): 2635-2647; WO 2016/172564 and WO 2016/170069), none of them involve a tankyrase inhibitor that selectively binds to the adenosine subsite of a tankyrase enzyme. Boddy et al. Regenerative Medicine, 2012; 7(6): 757-767 discloses a conditioned culture medium comprising IWR-1 and EGF.

### Summary

In vitro methods of using compounds that bind to Tankyrase 1 (TNKS1/PARP-5a/ARTD5) and tankyrase 2 (TNKS2/PARP-5b/ARTD6) are provided to induce the generation of PDX1+/NKX6-1+ pancreatic progenitor cells with increased capability to generate insulin-expressing cells.

In one aspect there is provided an in vitro method of producing PDX1+/NKX6-1+ pancreatic progenitor cells from a PDX1+ endodermal cell population, as further defined in the claims.

Other features and advantages of the present disclosure will become apparent from the following detailed description. It should be understood, however, that the detailed description and the specific examples while indicating preferred embodiments of the disclosure are given by way of illustration only, since various changes and modifications within the scope of the claims will become apparent to those skilled in the art from this detailed description.

### Brief description of the drawings

An embodiment of the present disclosure will now be described in relation to the drawings in which:

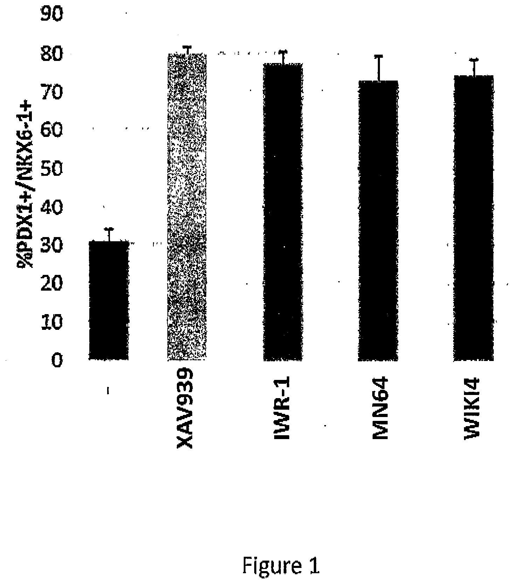

**Figure 1** shows that tankyrase inhibitors-treated cells induce the formation of or give rise to PDX1+/NKX6-1+ cells.

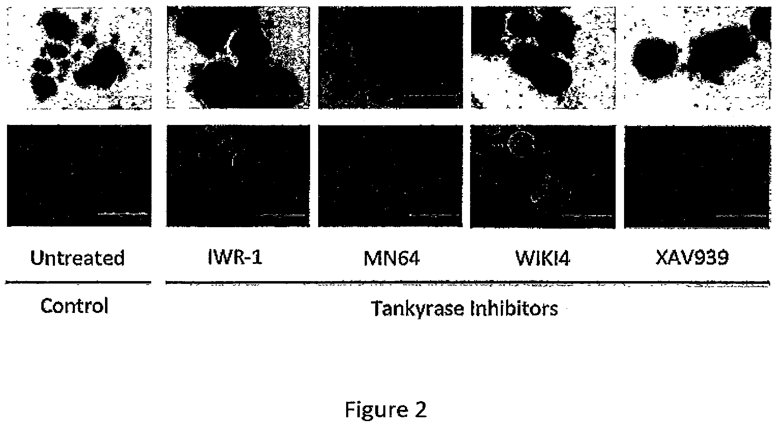

**Figure 2** shows that specific tankyrase inhibitors (IWR-1 and WIKI4) treated cells give rise to higher percentages of INSGFP+ cells (day 21) than cells treated with unspecific tankyrase inhibitors (XAV939 and MN64). Cells treated with various tankyrase pathway inhibitors (Cell treated with NE alone or NE in combination with tankyras inhibitors (NE + XAV939 1.5uM, NE + IWR-1-endo 3uM, NE + MN64 3uM, NE + WIKI4 9uM)) between day 8 and day 13 of differentiation were differentiated for an additional 10 days using a modified version of the differentiation protocol developed by Rezania and Pagliuca (Rezania et al 2014, Pagliuca 2014). Representative bright field and fluorescent images of cellular aggregates are shown at day 23 of differentiation. Results shown are from INS^{GFP/W} reporter line.

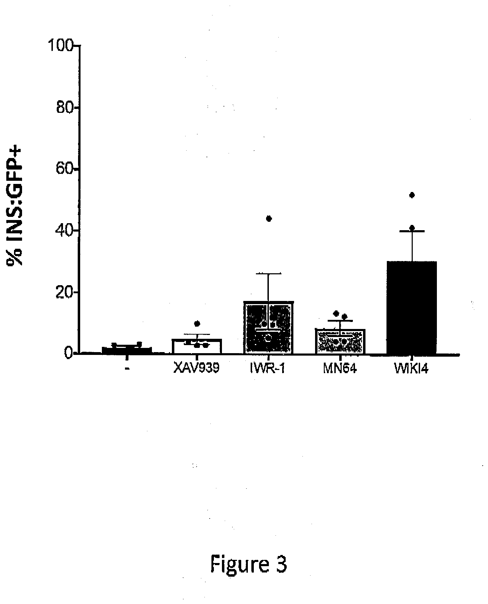

**Figure 3** Shown is a quantification of the results shown in Figure 2. Quantification of the percentage of INSULIN:GFP+ cells measured by flow cytometric analysis at day 23 of differentiation, arising from cells treated or untreated with different compounds: XAV939, IWR-1, MN64, WIKI4. Cells were treated using NE (-) or NE in combination with different compounds: XAV939 1.5uM, IWR-1 3uM, MN64 3uM, WIKI4 9uM. Results shown are from INS^{GFP/W} reporter line. Error bars represent standard deviation. n = 4.

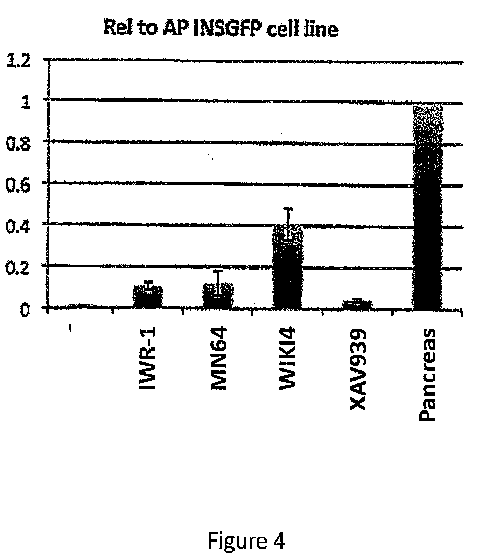

**Figure 4** shows that WIKI4 induce higher levels of INS mRNA compared to IWR, MN64 and XAV. Cells treated with different tankyrase inhibitors express different amounts of insulin transcript, as measured by QPCR. Shown is the relative expression of insulin transcript normalized to the housekeeping gene TBP (TATA Binding Protein) at day 23 of differentiation, where pancreatic progenitor cells were generated by treating cells with or without XAV939, IWR-1, MN64, WIKI4 (XAV939 1.5uM, IWR-1 3uM, MN64 3uM, WIKI4 9uM). Adult pancreas is used as positive control. Results shown are from INS^{GFP/W} reporter line. Error bars represent standard deviation. n = 3.

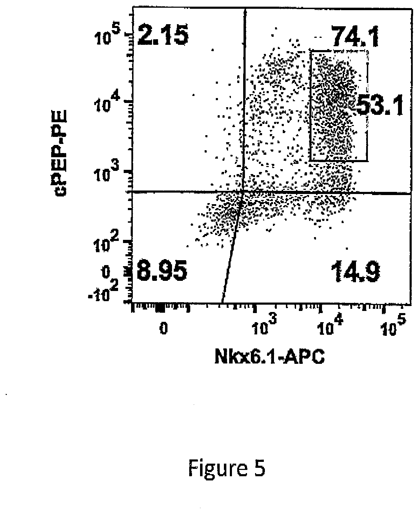

**Figure 5** shows that WIKI4-treatment (day 8-13) generates high percentages of C-Peptide+/NKX6-1+ insulin expressing beta-like cells using H1 cell line. Flow cytometry analysis for C-Peptide (a surrogate for insulin) and NKX6.1 performed at day 23 of differentiation, showing high percentage of C-peptide+/NKX6-1+ cells (beta-like cells). Numbers in each quadrant indicate the relevant percentage of the total population. Results shown are from H1 cell line.

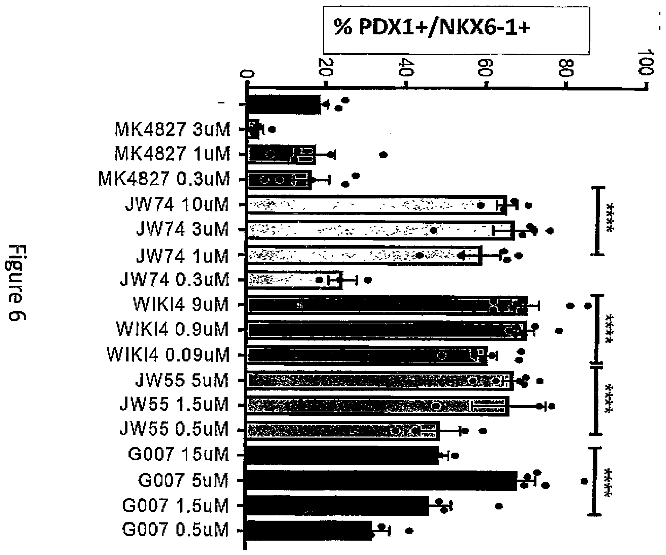

**Figure 6** shows specific tankyrase inhibitors generate high percentages of pancreatic progenitors as measured by PDX1/NKX6-1 double positive cells using H1 cell line. Specific tankyrase inhibitors, or ARTD inhibitors that specifically binding to ARTD5 and ARTD6 (TANK1 and TANK2), generate higher percentages of pancreatic progenitors as measured by PDX1/NKX6-1 double positive cells, but ARTD1/2 inhibitor (MK4827, a ARDT inhibitor that does not bind to the catalytic site of tankyrases) does not. Shown is a quantification of the percentage of PDX1+/NKX6.1+ cells measured by flow cytometric analysis at day 12 of differentiation, following no treatment (-) or treatment with MK4827, JW74, WIKI4, JW55, G007-LK from day 8 to day 12. Different concentrations of the selective inhibitors are used. Results shown are from H1 cells line at day 12 of differentiation. Error bars represent SEM. n ≥= 3.

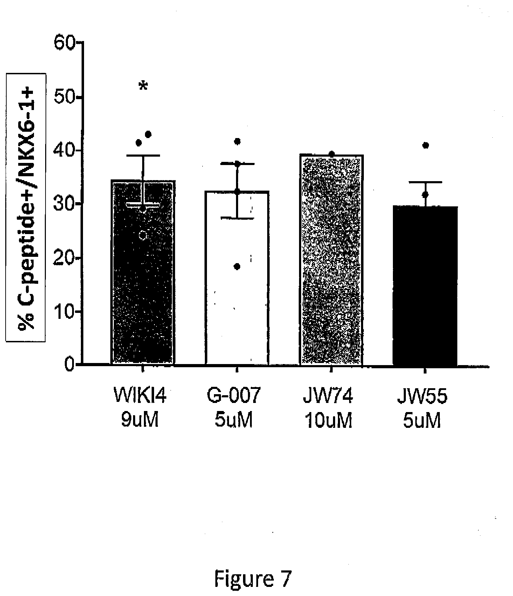

**Figure 7** shows specific tankyrase inhibitors generate high percentages of beta-like cells as measured by C-peptide/NKX6-1 double positive cells using H1 cell line. Cells treated with specific tankyrase inhibitors (WIKI4, JW74, JW55 and G007-LK) are able to generate a population of C-peptide+/NKX6.1+ insulin-expressing beta-like cells. Shown is a quantification of the percentage of C-Peptide+/NKX6.1+ cells measured by flow cytometric analysis at day 23 of differentiation, following treatment with WIKI4 (9uM), G007-LK (5uM), JW74 (10uM), JW55 (5uM) from day 8 to day 13. Results shown are from H1 cell line. Error bars represent SEM. n = 4 for all, n=1 for JW74.

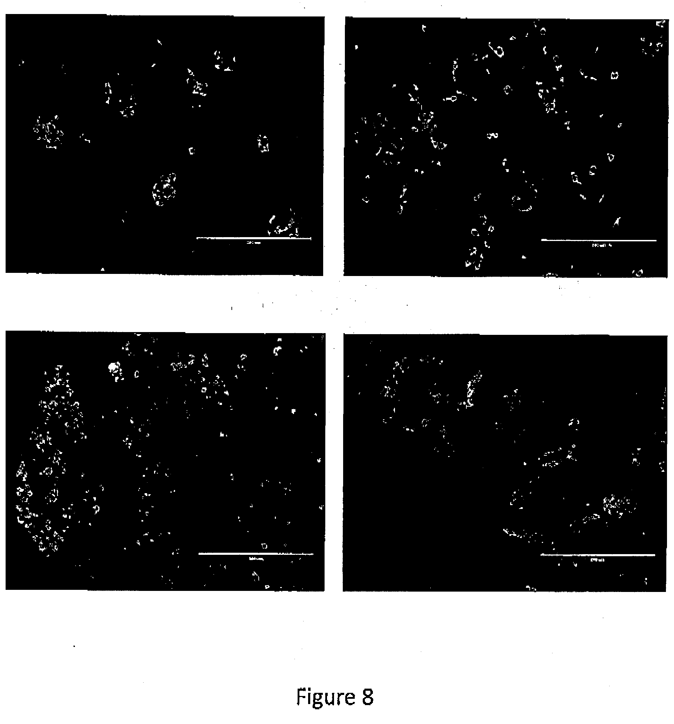

**Figure 8** shows immunofluorescence staining of cells, including insulin producing cells, differentiated from transplanted PDX1/NKX6-1 double positive progenitor cells produced according to methods described herein. WIKI4-generated pancreatic progenitors were transplanted subcutaneously in immunocompromised mice. Grafts were analyzed 3 months post-transplantation and stained for insulin, glucagon, cytokeratin 19 and trypsin. Immunofluorescence staining demonstrated that WIKI4-treated cells have the potential to develop into beta-like cells (Insulin+), alpha-like cells (Glucagon+), trypsin+ and cytokeratin19+ cells.

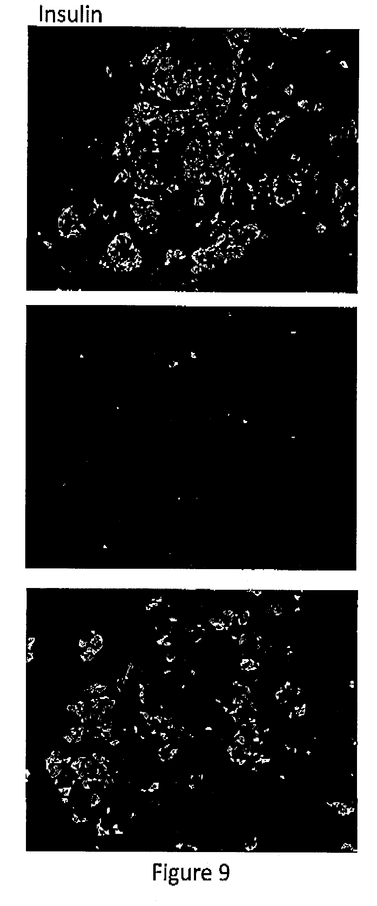

**Figure 9** shows immunofluorescence staining of cells, including insulin producing cells, differentiated from transplanted cultures containing C-Peptide/NKX6-1 double positive beta-like cells produced according to methods described herein. WIKI4-generated beta-like cells were transplanted subcutaneously and under the kidney capsule in immunocompromised mice. Grafts were analyzed 7.5 weeks post-transplantation and stained for insulin, Glucagon and somatostatin. Immunofluorescence staining demonstrated that WIKI4-treated cells have the potential to develop into beta-like cells (Insulin+) alpha-like cells (Glucagon+), delta-like cells (somatostatin+).

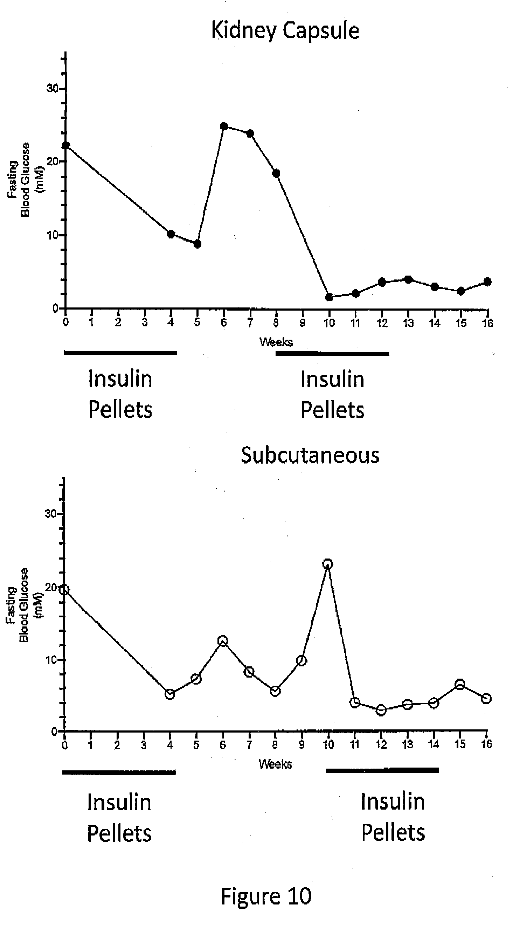

**Figure 10** shows normalization of glycemia after transplantation of cultures containing C-Peptide/NKX6-1 double positive beta-like cells produced according to methods described herein. WIKI4-generated beta-like cells were transplanted subcutaneously and under the kidney capsule in immunocompromised mice. Immunocompromised mice were rendered hyperglycemic with streptozotocin injection. Blood glucose was monitored weekly and normoglycemia (in the absence of insulin pellets) was observed from week 12.

### Detailed description of the Disclosure

Described herein is the application of tankyrase inhibitors for effectively generating pancreatic progenitor cells, which have the potential to generate a high percentage of insulin-producing beta-like cells. The addition of compounds, such as WIKI4, JW74, JW55, and G007-LK, following PDX1 expression lead to the formation of a greater percentage of PDX1+/NKX6-1+ cells that can give rise to insulin producing beta-like cells in vitro. As a result, this approach provides an alternative and efficient protocol for the generation of insulin-producing cells from different hESC and hiPSC lines.

Tankyrases belong to the Diphtheria toxin-like ADP-ribosyltransferase (ARTD) enzyme superfamily that comprises 17 members in humans. They catalyze the transfer of an ADP-ribose from the co-substrate NAD+ on a target protein. This covalent posttranslational modification leads to the attachment of one or multiple ADP-ribose (ADPr) molecules to the target protein. Tankyrase 1 (TNKS1/PARP-5a/ARTD5) and tankyrase 2 (TNKS2/PARP-5b/ARTD6) belong to the polymer forming class of this enzyme family (ARTD1-6), but they have a unique domain organization separating them from the other members. In addition to the catalytic ARTD domain located at the C-terminus, they contain a sterile alpha motif (SAM) next to the catalytic domain, which is responsible for the multimerization of the tankyrases. Additionally, they contain five ankyrin repeat clusters (ARC) that confer specificity to specific proteins.

The catalytic domain of tankyrases is characterized by two domains: the donor site and the acceptor site. The donor site, where NAD+ naturally binds to, can be divided into two subsites: the nicotinamide and the adenosine subsites.

Applicant demonstrates that the highest percentage of insulin-producing cells is generated by using specific ARTD inhibitors with selective activity to tankyrases and minimal or no binding to other ARTD enzymes. Commercially available ARTD inhibitors can bind with different specificity to the donor site. A select group of inhibitors have been designed to specifically bind to the adenosine subsite only and have been shown specificity to TANK inhibition, as oppose to other ARTD enzymes.

Tankyrase inhibition following PDX1 expression give rise to cells that express PDX1 and NKX6-1 and have the potential to generate insulin-expressing cells using published differentiation protocols (Rezania 2014, Pagliuca 2014).

PDX1 expression may be achieved at day 7-8 of differentiation of hESCs and hiPSCs and tankyrase inhibitors are administered for six days (day 12-13).

Insulin expression may be induced by culturing day 12-13 cells in the presence of thyroid hormone, Alk5 inhibitors, retinoic acid, BMP inhibition, sonic hedgehog inhibition as previously described (Rezania 2014, Pagliuca 2014). Insulin expression can be detected from day 16 onwards.

The pancreatic progenitors generated using tankyrase inhibitors could be used, without limitation, for transplantation for clinical purposes into humans and other species, modeling normal pancreatic development *in vitro,* utilizing patient-specific hiPSCs to model diseases, and drug discovery and testing for pancreas-associated diseases.

An aspect of the present disclosure includes an in vitro method of producing PDX1+/NKX6-1+ pancreatic progenitor cells from a PDX1+ population,as further defined in the claims.

As used herein, a "tankyrase inhibitor" is a molecule, compound, or composition that inhibits the enzymatic activity of tankyrase enzymes. Tankyrase inhibitors can bind to the nicotinamide and/or the adenosine subsite of tankyrase enzymes. A tankyrase inhibitor that binds to the adenosine subsite of tankyrase enzymes may also bind to the nicotinamide subsite to varying degrees. Some tankyrase inhibitors that bind to the adenosine subsite exhibit equal or higher affinity to the adenosine subsite over the nicotinamide subsite. While preferred tankyrase inhibitors selectively bind to the adenosine subsite. Examples of tankyrase inhibitors include, but are not limited to: XAV939, IWR-1-endo, MN64, WIKI4, G007-LK, JW74, or JW55. Other examples of tankyrase inhibitors are provided, for example in Anumala et al. (2017).

In the invention, a tankyrase inhibitor binds to a catalytic site of tankyrase enzymes. As used herein, the term "tankyrase enzyme" includes without limitation, tankyrase 1 (TNK1) and tankyrase 2 (TNK2). The tankyrase inhibitor binds to the adenosine subsite of TNK1 and TNK2, as further defined in the claims. Certain tankyrases enzymes are known in the art and discussed, for example in **Riffell et al. (2012).**

Inthe invention, the tankyrase inhibitor binds selectively to the adenosine subsite of tankyrase enzymes and has a higher affinity to the adenosine subsite than the nicotinamide subsite.. Examples of tankyrase inhibitors that bind to the adenosine subsite include, but are not limited to, WIKI4, G007-LK, JW55, JW74, CMP24, CMP40, and CMP4. Preferably the tankyrase inhibitor is WIKI4, G007-LK, JW55, or JW74.

Examples of tankyrase inhibitors that bind to both the adenosine and nicotinamide subsites of tankyrase enzymes include, but are not limited to NVP-TNKS656, CMP4b, and compound 16.

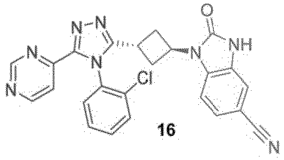

In some embodiments, the tankyrase inhibitor does not bind to the nicotinamide subsite of tankyrase enzymes.

In an embodiment, the in vitro method further comprises contacting the PDX1+ endodermal cell population with a BMP inhibitor component.

The term "contacting" (e.g. contacting an endodermal cell population with a component or components) refers to any suitable means by which the cell is cultured or incubated with the component(s) together in vitro. For example, the compound is added to the cells in culture, or is transferred to or mixed with culture medium containing the compound. For example the cells may be treated in adherent culture, or in suspension culture, the components can be added temporally substantially simultaneously (e.g. together in a cocktail) or sequentially (e.g. within 1 hour from an addition of a first component). The cells can also be contacted with another agent such as a growth factor or other differentiation agent or environments to stabilize the cells, or to differentiate the cells further and include culturing the cells under conditions known in the art for example for culturing the pluripotent (and/or differentiated) population.

The "endodermal cell population" as used herein refers to a population of cells comprising at least 20%, at least 30%, at least 40%, at least 50%, at least 60%, at least 70%, at least 80%, at least 90% PDX1+ cells. Endoderm cells correspond for example to an embryonic posterior foregut cell population, which express at least PDX1. Other factors can also be expressed. The population can be NKX6-1 negative (NKX6-1-) or express low levels of NKX6-1. The endodermal cell population can for example be identified by flow cytometric and molecular analysis for one or more markers such as PDX1. The endodermal cell population can for example be in a 2D (monolayer) or 3D (Embryoid Body or other form of aggregates) formats.

The term "endoderm" as used herein refers to the innermost of the three primary germ cell layers in the very early embryo. The other two germ cell layers are the ectoderm (outer layer) and the mesoderm (middle layer), the endoderm being the innermost layer. The endoderm forms into some internal organs and the epithelial linings of the digestive and respiratory systems. An endoderm cell differentiates to give rise first to the embryonic gut and then to the lining of the gut (esophagus, stomach, intestine, rectum, colon), pharyngeal pouch derivatives (tonsils, thyroid, thymus, parathyroid glands), lung, liver, gall bladder and pancreas.

The term "pluripotent stem cell" as used herein refers to a cell that has the capacity to self-renew by dividing, and to develop or differentiate, under different conditions, to more than one differentiated cell type, for example into one or more cell types characteristic of the three germ cell layers, and includes embryonic stem cells and induced pluripotent stem cells. Embryonic stem cells and induced pluripotent stem cells are examples of pluripotent stem cells. Pluripotent cells are characterized by their ability to differentiate to more than one cell type using, for example, a nude mouse teratoma formation assay. Pluripotency is also evidenced by the expression of embryonic stem (ES) cell marker.

The term "progenitor cell" refers to cells that have a cellular phenotype that is at an earlier step along a developmental pathway or progression than is a fully differentiated cell relative to a cell which it can give rise to by differentiation. Progenitor cells can give rise to multiple distinct differentiated cell types or to a single differentiated cell type, depending on the developmental pathway and on the environment in which the cells develop and differentiate.

The term "pancreatic progenitor cell" refers to a cell which is capable of forming any of: pancreatic endocrine cells (such as glucagon producing alpha cells, insulin producing beta cells, somatostatin producing delta cells, ghrelin producing epsilon cells and pancreatic polypeptide producing cells), or pancreatic acinar cells (e. g. amylase producing- and/or trypsin producing-pancreatic cells) or pancreatic ductal cells. Formation or development of one more of pancreatic endocrine cells, pancreatic acinar cells or pancreatic ductal cells may be induced by addition of components such as a combination of a thyroid hormone, alk5i, retinoic acid, BMP and SHH inhibition and/or resulting from the environment in which the progenitor cell develops. Similarly injection of PDX1+/NKX6-1+ pancreatic progenitor cells in vivo can result in development of endocrine, acinar and ductal cells.

The term "functional beta cell" as used herein means a type of pancreatic cell, which in the pancreas is predominantly located in areas called the islets of Langerhans, that makes and releases insulin.

The term "insulin-producing beta-like cell" as used herein refers to a cell that makes insulin and has at least one or more, at least two or more, or at least three or more characteristics of functional beta cells, or pancreatic cells which in the pancreas is located in areas called the islets of Langerhans.

The term "stem cell" as used herein, refers to an undifferentiated cell that can differentiate into specialized cells. A stem cell is capable of proliferation, self-renewal and giving rise to more stem cells or progenitor cells having the ability to generate a large number of mother cells that can in turn give rise to differentiated or differentiable daughter cells. The daughter cells can for example be induced to proliferate and produce progeny that subsequently differentiate into one or more mature cell types, while also retaining one or more cells with parental developmental potential.

In the context of a cell, the term "differentiated", or "differentiating" is a relative term and a "differentiated cell" is a cell that has progressed further down the developmental pathway than the cell it is being compared with, and in some cases a "differentiated cell" is more specialized. In some cases, a "differentiated cell" is a cell that changed from one cell type to another. Thus, stem cells can differentiate to lineage-restricted precursor cells (such as an endodermal progenitor cell), which in turn can differentiate into other types of precursor cells further down the pathway and then to an end-stage differentiated cell, which plays a characteristic role in a certain tissue type, and may or may not retain the capacity to proliferate further.

The term "embryonic stem cell" is used to refer to the pluripotent stem cells that can be obtained from the inner cell mass of blastocysts. Non-human blastocysts can be derived from non-human somatic cell nuclear transfer (see, for example, U.S. Pat. Nos. 5,945,577, 5,994,619, 6,235,970). The distinguishing characteristics of an embryonic stem cell define an embryonic stem cell phenotype. Accordingly, a cell has the phenotype of an embryonic stem cell if it possesses one or more of the unique characteristics of an embryonic stem cell such that that cell can be distinguished from other cells. Exemplary distinguishing embryonic stem cell characteristics include, without limitation, gene expression profile, proliferative capacity, differentiation capacity, karyotype, responsiveness to particular culture conditions, and the like.

The term "expression" refers to the cellular processes by which information from a gene or information encoded in a nucleic acid molecule is used in the synthesis of an "expression product". The processes involve producing RNA and proteins and as appropriate, secreting proteins, including where applicable, but not limited to, for example, transcription, translation, folding, modification and processing. "Expression products" include RNA transcribed from a gene and polypeptides obtained by translation of mRNA transcribed from a gene.

The term "PDX1+/NKX6-1+ positive pancreatic progenitor cell" as used herein refers to a cell which is derived for example from a pancreatic endoderm cell and which has the capacity to differentiate at least into insulin producing cells, such as pancreatic beta cells. A PDX1+/NKX6-1+ pancreatic progenitor expresses the markers PDX1 and NKX6-1 and can express for example increased levels of PTF1A and SOX9 compared to a pluripotent stem cell (PSC).

The term "NKX6-1+" as used herein refers to the mRNA (and/or optionally cDNA) or protein product of the "NK6 homeobox 1" gene (Gene ID: 4825), the sequences, including protein and mRNA sequences (and/or optionally cDNA).

The term "PDX1" as used herein refers to the mRNA (and/or optionally cDNA) or protein product of the "Pancreatic and duodenal homeobox 1" gene (Gene ID: 3651), the sequences, including protein and mRNA sequences (and/or optionally cDNA).

The term "PDX1+/NKX6-1+ cell population" as used herein refers to a population of cells comprising at least 10%, at least 15%, at least 20%, at least 25%, at least 30%, at least 35%, at least 40%, at least 50%, at least 60%, at least 70%, at least 80% or up to about 95% NKX6-1+ cells. Other factors can also be expressed. The NKX6-1 positive cell population can also comprise at least 30%, 35%, 40% or at least 50%, at least 60%, at least 70%, at least 80% or up to about 95% NKX6-1+ pancreatic progenitor cells. The NKX6-1 positive cell population can for example be in a 2D (monolayer) or 3D (Embryoid Body or other form of aggregates) formats.

In some embodiments, contacting with EGF and a tankyrase inhibitor that selectively binds to the adenosine subsite of tankyrase enzymes gives rise to PDX1+/NKX6-1+ cells, which are increased by the addition of a BMP inhibitor. In some embodiments, the BMP inhibitor is noggin, Dorsomorphin, LDN, CHORDIN, BMPR1A, or BMPR1B. In one embodiment, the BMP inhibitor is noggin.

The term "BMP inhibitor component" as used herein means any polypeptide or compound that inhibits TGF-beta/BMP family signal transduction for example by binding a TGF beta family ligand, including but not limited to agents provided in Example 10, including for example Noggin (NOG) such as human Noggin having Gene Identification number **(Gene ID: 9241)** as well as active conjugates and fragments thereof, including naturally occurring active conjugates and fragments; bone morphogenetic protein receptor, type IA (BMPR1A), for example human BMPR1A having for example Gene Identification number **(Gene ID: 657)** as well as active conjugates and fragments thereof, including naturally occurring active conjugates and fragments; bone morphogenetic protein receptor, type IB (BMPR1B), for example human BMPR1B having for example Gene Identification number **(Gene ID: 658)** as well as active conjugates and fragments thereof, including naturally occurring active conjugates and fragments; small molecule chemical inhibitor Dorsomorphin and salts, solvates and combinations thereof; small molecule chemical inhibitor LDN 193189 also known as "DM-3189" and/or salts and/or solvates thereof; and CHORDIN, for example human CHORDIN (CHRD) having for example Gene Identification number **(Gene ID: 8646)** as well as active conjugates and fragments thereof, including naturally occurring active conjugates and fragments; and/or combinations of two or more of the above, including for example combinations comprising Noggin. The sequences, including protein and mRNA sequences, of each (e.g. each component identified by Gene ID).

The term "Noggin" as used herein refers to a bone morphogenetic protein antagonist having Gene Identification number **(Gene ID: 9241),** the sequences, including protein and mRNA sequences.

The compound "Dorsomorphin" includes compounds of the formula: 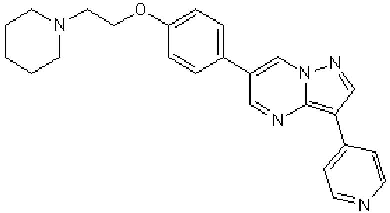 salts, solvates and/or combinations thereof.

The compound "LDN" means compounds having the formula: 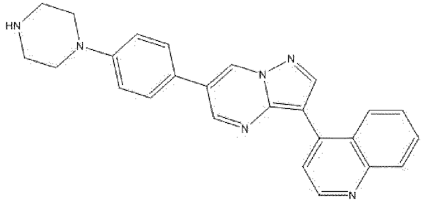 salts, solvates and/or combinations thereof.

In an embodiment, the Noggin component is Noggin. The concentration of Noggin can for example range from about 1 ng to about 500 ng/ml for example from about 1 ng to about 250 ng/ml, from about 10 ng to about 250 ng/ml from about 10 ng to about 100 ng/ml. In another embodiment, the Noggin concentration is about 10 ng/ml, about 20 ng/ml, about 30 ng/ml, about 40 ng/ml, about 50 ng/ml, about 60 ng/ml, about 70 ng/ml, about 80 ng/ml, about 90 ng/ml, about 100 ng/ml, about 150 ng/ml, about 200 ng/ml, about 300 ng/ml, about 400 ng/ml, or about 500 ng/ml.

The term "EGF component" as used herein means any polypeptide or small molecule that activates the EGF receptor family memberErbB1/HER-1/EGFR, gene ID:1956, and optionally any of the EGF receptor family members ErbB2/HER-2/neu, gene ID:2064 ErbB3/HER-3, gene ID: 2065, and/or ErbB4/HER-4, gene ID:2066) including for example but not limited to epidermal growth factor (EGF), for example human EGF having for example Gene Identification number **(Gene ID: 1950)** as well as active conjugates and fragments thereof, including naturally occurring active conjugates and fragments; Amphiregulin (AR) for example human AR/AREG having for example Gene Identification number **(Gene ID: 374)** as well as active conjugates and fragments thereof, including naturally occurring active conjugates and fragments; Transforming Growth Factor α (TGFα) for example human TGFα having for example Gene Identification number **(Gene ID: 7039),** as well as active conjugate and fragments thereof, including naturally occurring active conjugates and fragments; Betacellulin (BTC) for example human BTC having for example Gene Identification number **(Gene ID: 685)** as well as active variants and fragments thereof, including naturally occurring active conjugates and fragments; Heparin-binding EGF-like growth factor (HB-EGF) having for example Gene Identification number **(Gene ID: 1839),** as well as active conjugate and fragments thereof, including naturally occurring active conjugates and fragments; Epiregulin (EREG/ER) for example human EREG having for example Gene Identification number **(Gene ID: 2069),** as well as active variants and fragments thereof, including naturally occurring active variants and fragments; Neuregulins (including for example NRG1, NRG2, NRG3 NRG4) for example human NRGs having for example Gene Identification number **(Gene ID: 3084, Gene ID: 9542, Gene ID: 10718, Gene ID: 145957,** respectively), as well as active variants and fragments thereof, including naturally occurring active variants and fragments and/or combinations of two or more of the above, for example a combination comprising human EGF.

In an embodiment, the term "EGF" as used herein refers to Epidermal growth (EGF), for example human EGF having for example Gene Identification number **(Gene ID: 1950)** as well as active conjugates and fragments thereof, including naturally occurring active conjugates and fragments.

In an embodiment, the EGF component, i.e. the agent that activates ErbB1, is EGF. The concentration of EGF can for example range from about 1 ng to about 500 ng/ml for example from about 1 ng to about 250 ng/ml, from about 10 ng to about 250 ng/ml from about 10 ng to about 100 ng/ml. In another embodiment, the EGF concentration is about 10 ng/ml, about 20 ng/ml, about 30 ng/ml, about 40 ng/ml, about 50 ng/ml, about 60 ng/ml, about 70 ng/ml, about 80 ng/ml, about 90 ng/ml, about 100 ng/ml, about 150 ng/ml, about 200 ng/ml, about 300 ng/ml, about 400 ng/ml, or about 500 ng/ml.

The tankyrase inhibitor is WIKI4, G007-LK, JW74, JW55, CMP24, CMP40, or CMP4, or a salt, solvate and/or conjugate thereof. In one embodiment, the tankyrase inhibitor is WIKI4, G007-LK, JW74 or JW55 or a salt, solvate and/or conjugate thereof. The concentration of the tankyrase inhibitor can for example range from about 1 µM to about 100 mM for example from about 10 µM to about 50 mM, from about 1 mM to about 100 mM, or from about 1 mM to about 50 mM. In another embodiment, the Wnt inhibitor concentration is about 1 uM, about 2 uM, about 5 uM, about 10 uM, about 15 uM, about 20 uM, about 30 uM, about 40 uM, about 50 uM, about 60 uM, about 70 uM, about 80 uM, about 90 uM, or about 100 uM, about 1 mM, about 2 mM, about 5 mM, about 10 mM, about 15 mM, about 20 mM, about 30 mM, about 40 mM, about 50 mM, about 60 mM, about 70 mM, about 80 mM, about 90 mM, or about 100 mM.

In one embodiment, the in vitro method comprises contacting the endodermal cell population with at least one BMP inhibitor component (such as noggin); at least one EGF component (i.e. an agent that activates ErbB1) and at least one tankyrase inhibitor that selectively binds to the adenosine subsite of tankyrase enzymes.

In another embodiment, the in vitro method comprises contacting the endodermal cell population with a BMP inhibitor component (such as noggin), a EGF component (i.e. an agent that activates ErbB1), and a tankyrase inhibitor comprising WIKI4, G007-LK, JW74 or JW55.

In another embodiment, the in vitro method comprises contacting the endodermal cell population with at least one BMP inhibitor component (such as noggin), at least one EGF component (i.e. at least one agent that activates ErbB1), and at least one tankyrase inhibitor comprising at least one of WIKI4, G007-LK, JW74 and JW55.

In an embodiment, the endodermal cell population is contacted with the combination of at least one BMP inhibitor component, at least one EGF component (i.e. at least one agent that activates ErbB1), at least one tankyrase inhibitor, that selectively binds to the adenosine subsite of tankyrase enzymes, as further defined in the claims, for at least or about 3 days, at least about 4 day, at least about 5 day, at least about 6 day, at least about 7 day, at least about 8 day, at least about 9 day, or at least about 10 days. In another embodiment, the endodermal cell population is contacted with the combination for at least about 11 days, at least about 12 days, at least about 13 days, at least about 14 days, or at least about 15 days. It is found that PDX1+/NKX6-1+ positivity and/or capacity to generate insulin producing cells can persist for example at least 30 days or more in cultured cells.

In an embodiment, the in vitro methods described herein and as further defined in the claims induce the production of greater than about 10%, 20%, 25%, 30%, 35%, 40%, 45%, 50%, 55%, 60%, 65%, 70%, 75%, 80%, 85%, 90%, or about 95% PDX1/NKX6-1 double positive pancreatic progenitor cells from a population of endodermal PDX1+ cells and/or for example greater than about 5%, 10%, 15%, 20%, 25%, 30% 35%, 40%, 45%, 50%, 55%, 60%, 65%, 70%, 75%, 80%, 85%, 90%, or about 95% insulin producing cells from PDX1/NKX6-1 double positive pancreatic progenitor cells.

In an embodiment, the in vitro methods using tankyrase inhibitors that selectively bind to the adenosine subsite of tankyrase enzymes, as further defined in the claims, induce the production of greater than about 25%, 30% 35%, 40%, 45%, 50%, 55%, 60%, 65%, 70%, 75%, 80%, 85%, 90%, or about 95% insulin producing cells from PDX1/NKX6-1 double positive pancreatic progenitor cells.

Differentiation can be detected by determining the level of pancreatic progenitor markers. For example, NKX6-1, PDX1, PTF1A, and SOX9 are pancreatic progenitor markers whose mRNA expression can be detected for example by RT-PCR. Differentiation can also be detected using antibodies that recognize pancreatic progenitor cells, such as antibodies against GP2, CD142 and anti-HPx1 and anti-HPx2 (Kelly et al., 2011, Ameri 2017, Cogger 2017, Ramond 2017).

In an embodiment, the endodermal cell population is differentiated from pluripotent stem cells (PSCs) such as an embryonic stem cell (ESC) or an induced pluripotent stem cells (iPSCs).

In an embodiment, the pluripotent stem cell is from a mammal, such as a human. In an embodiment, the pluripotent stem cell is a human ESC (hESC) or a human iPSC (hiPSC).

As used herein, the terms "iPSC" and "induced pluripotent stem cell" are used interchangeably and refers to a pluripotent stem cell artificially derived (e.g., induced or by complete reversal) from a non-pluripotent cell, typically an adult somatic cell, for example, by inducing expression of one or more genes (including POU4F1/OCT4 (Gene ID; 5460) in combination with, but not restricted to, SOX2 (Gene ID; 6657), KLF4 (Gene ID; 9314), cMYC (Gene ID; 4609), NANOG (Gene ID; 79923), LIN28/ LIN28A (Gene ID; 79727)).

In an embodiment, the method as further defined in the claims comprises steps for obtaining the endodermal cell population. For example, as described herein differentiation from a pluripotent stem cell such as an ESC or an iPSC to an endodermal cell involves a series of steps that is characterized in Figure 1 of WO 2013/163739 as comprising Stages 1 to 3. Stage 1 can further be divided into substages for example three substages spanning for example days 0 to 5. Stage 2 can span for example 2 to 3 days and stage 3 can span for example 1 to 4 days. As described in Nostro et al 2011, Stage 1 can comprise contacting a population of pluripotent stems cells with ActA (or other Nodal agonist) and Wnt3a at day 0, contacting the population with Act A, bFGF and optionally Wnt3a at day 1 and contacting the population with ActA and bFGF at day 2 to produce Stage 1 differentiated cells. Stage 2 for example can include contacting the population to be differentiated (e.g. the Stage 1 differentiated cells) with Fibroblast Growth Factor 10 (FGF10), optionally wingless-type MMTV integration site family member 3A (Wnt3a) and optionally Dorsomorphin to produce Stage 2 differentiated cells. Stage 3 for example can include contacting the population to be differentiated (e.g. the Stage 2 differentiated cells) with Noggin, optionally cyclopamine-KAAD (Cyc) (or any other hedgehog (HH) signaling inhibitors), Retinoic acid (RA), optionally FGF10 to provide an endodermal cell population. Dorsomorphin can be substituted for example with other noggin components such as chordin LDN 193189 and BMPRs; ActA can be substituted with other Nodal agonists and Wnt 3a can be substituted with other Wnt signaling agonists such as or other wnt/beta catenin agonist such as CHIR99021. Similarly, bFGF and FGF10 can be substituted with other FGFs or compounds that activate the same receptor as bFGF or FGF10 respectively. Retinoic acid can be substituted for example by a retinoic acid analog. The protocol can for example be the protocol previously described (Nostro et al., 2011), and/or with modifications (e.g. addition of Wnt3a at day 1 and Exendin-4, Ex-4 from day 5 to day 7). Variations include for example, omitting FGF10 and/or Exendin-4 from the protocol and using for example CHIR99021 (Stemgent 04-0004) as replacement for Wnt3a. Protocols for generating Stage 3 cells are described for example in US Patents 7,989,204, 7,993,916, 8,129,182 and 8,187,878.

ActA is a nodal agonist. The term "nodal agonist" as used herein means any molecule that activates nodal signal transduction such as "nodal" (for example human nodal such as Gene ID: 4338) or "activin". The term "activin" or "ActA" as used herein refers to "Activin A" ( for example Gene ID: 3624), for example human activin, as well as active conjugates and fragments thereof, optionally including naturally occuring active conjugates and fragments, that can for example activate nodal signal transduction as well as active conjugates and fragments thereof, including naturally occuring active conjugates and fragments.

Wnt3A is a wnt signaling agonist. The term "a wnt signaling agonist" as used herein means any molecule that activates wnt/beta-catenin receptor signaling in a hepatocyte and incldues for example Wnt3a and as well as GSK3 selective inhibitors such as CHIR99021 (Stemolecule^{™} CHIR99021 Stemgent), 6-Bromolndirubin-3'-Oxime (BIO) (Cayman Chemical (cat:13123)), or Stemolecule^{™} BIO from Stemgent (cat:04003). CHIR99021 is a selective inhibitor of GSK3. The GSK3 selective inhibitors contemplated are for example selective inhibitors for GSK-3α/β in the Wnt signaling pathway.

FGF10 and bFGF are FGF members that activate FGF receptor signaling.

The term "FGF agonist" as used herein means a molecule such as a cytokine, including for example FGF, or a small molecule, that activates a FGF signalling pathway, e.g binds and activates a FGF receptor. The term "FGF" as used herein refers to any fibroblast growth factor, for example human FGF1 (Gene ID: 2246), FGF2 (also known as bFGF; Gene ID: 2247), FGF3 (Gene ID: 2248) , FGF4 (Gene ID: 2249), FGF5 (Gene ID: 2250), FGF6 (Gene ID: 2251), FGF7 (Gene ID: 2252), FGF8 (Gene ID: 2253), FGF9 (Gene ID: 2254) and FGF10 (Gene ID: 2255) optionally including active conjugates and fragments thereof, including naturally occuring active conjugates and fragments. In certain embodiments, FGF is bFGF, FGF10, FGF4 and/or FGF2.

Exemplary ranges for added components for differentiating pluripotent stem cells up to stage 3 (e.g. d7) are provided as previously described (Nostro et al., 2011), and/or cytokines and small molecules can be used at the following concentrations: Activin A (Act1: 10-1000 ng/ml), Wnt3a (Stage 1: 25 ng/ml), CHIR99021 (Stage 1: 0.1-3 µM), bFGF (0.1-50 ng/ml), FGF10 (5-500 ng/ml), Wnt3a (Stage 2: 3 ng/ml), Dorsomorphin (DM: 0.25-0.75 µM), Noggin (NOG: 1-500 ng/ml), Cyclopamine-KAAD (Cyc: 0.5-2.5 µM), Retinoic Acid (RA: 0.02-2 µM) EGF (1-500 ng/ml), Wnt inhibitor (1uM-100mM).

Other in vitro methods of differentiating cells to obtain an endodermal cell population can also be used.

A further aspect includes an in vitro method of producing PDX1+/NKX6-1+ pancreatic progenitor cells from a pluripotent stem cell population, the method comprising one or more of steps (or substeps) a, b and c together with step d, the steps comprising:
a. contacting a pluripotent stem cell population with a combination of:
   I. a nodal agonist, optionally ActA and a wnt signaling agonist, optionally, Wnt3a or CIHR 99021,
   II. a nodal agonist, optionally Act A, a FGF agonist, optionally bFGF and optionally a wnt signaling agonist, optionally Wnt3a CIHR 99021,; and
   III. a nodal agonist, optionally ActA and a FGF agonist, optionally bFGF;
   to produce Stage 1 differentiated cells;
b. contacting the Stage I differentiated cells with a FGF agonist, optionally FGF10, and optionally wnt signaling agonist, optionally Wnt3a and/or a noggin component, optionally Dorsomorphin, to produce Stage 2 differentiated cells;
c. contacting the Stage 2 differentiated cells with a Noggin component, optionally Noggin, Retinoic acid (RA) or RA analog, and optionally cyclopamine-KAAD (Cyc), a FGF agonist, optionally FGF10 and/or an Exendin-4 component, optionally Exendin-4 to provide a endodermal cell population; and
d. contacting the PDX1+ endodermal cell population with an EGF component (i.e. an agent that activates ErbB1) and a tankyrase inhibitor that selectively binds to the adenosine subsite of a tankyrase enzyme, in an amount sufficient to induce the differentiation of at least a portion of the endodermal cell population into PDX1+/NKX6-1+ pancreatic progenitor cells, as further defined in the claims. In some embodiments, step d) further comprising contacting the PDX1+ endodermal cell population with a BMP inhibitor component.

In an embodiment, Stage 2 (optionally instead of Dorsomorphin) and/or further comprises contacting the population with at least one Noggin component.

In an embodiment, the steps comprise two or more of steps of steps a, b and c. For example, a person skilled in the art would recognize that the method to be applied to a Stage 2 cell or equivalent thereof would include steps c) and d). Similarly, one or more substeps of a) can be performed and/or excluded depending on the substage of the starting cell population.

A "Stage 1 cell" as used herein means an endoderm cell characterized at least by the expression of SRY-box containing gene 17 (SOX17) (Gene ID:64321) and chemokine (C-X-C motif) receptor 4 (CXCR4) (Gene ID:7852) generated following in vitro differentiation of human pluripotent stem cells (hESCs and hiPSCs) in 2D or 3D formats, as described for example in Nostro et al., 2011.

A "Stage 2 cell" as used herein means an endoderm cell characterized at least by the expression of forkhead box A2 (FOXA2) (Gene ID:3170) and by the expression of HNF1 homeobox B (HNF1B) (Gene ID:6928) generated following in vitro differentiation of human pluripotent stem cells (hESCs and hiPSCs) in 2D or 3D formats, for example as described in Nostro et al., 2011.

A "Stage 3 cell" as used herein means an endoderm cell characterized at least by the expression of PDX1 (Gene ID: 3651) generated following in vitro differentiation of human pluripotent stem cells (hESCs and hiPSCs) in 2D or 3D formats, for example as described in Nostro et al., 2011.

A "Stage 4 cell" as used herein means an endoderm cell characterized at least by the expression of NK6 homeobox 1 (NKX6-1) (Gene ID:4825) and by the expression of pancreatic and duodenal homeobox 1 (PDX1) (Gene ID:3651) generated following in vitro differentiation of human pluripotent stem cells (hESCs and hiPSCs).

The PDX1/NKX6-1 double positive progenitor cells can for example be used to generate insulin producing cells in vivo and/or in vitro.

For example, transplanted PDX1/NKX6-1 double positive progenitor cells produced according to a method described herein differentiate to insulin producing cells (Figure 8).

Detection of insulin producing cells can include for example detection of c-peptide positivity for example by flow cytometry and/or insulin and/or glucagon expression for example by RT-PCR.

As used herein, the term "insulin producing cell" refers to a cell differentiated from a pancreatic progenitor, which secretes insulin. An insulin producing cell includes functional pancreatic beta-cells, as well as pancreatic beta-like cells that synthesize, express, or secrete insulin in a constitutive or inducible manner. A population of insulin producing cells, e.g. produced by differentiating endodermal cells to pancreatic progenitors and subsequent differentiation into insulin producing cells according to the methods described herein, can be functional pancreatic beta-cells or beta-like cells (e.g., cells that have at least two characteristics of an endogenous functional beta-cell). It is also contemplated that the population of insulin producing cells, e.g. produced by the methods as disclosed herein, can comprise pancreatic beta-cells or pancreatic beta-like cells, and can also contain non-insulin producing cells (e.g. cells with a beta-cell like phenotype with the exception that they do not produce or secrete insulin).

Differentiation of PDX1/NKX6-1 double positive progenitor cells into insulin producing cells is performed by, for example, the methods disclosed in (Rezania 2014, Pagliuca 2014).

As used herein, "C-peptide positive/ NKX6-1 positive cell" refers to a cell which is derived for example from a pancreatic endoderm cell, and expresses the markers NKX6-1 and C-peptide. As used herein, "C-peptide" refers to is a short 31-amino-acid polypeptide that connects insulin's A-chain to its B-chain in the proinsulin molecule, or a variant of this polypeptide, or a fragment of this polypeptide. C-peptide is a marker for insulin production. C-peptide positive/ NKX6-1 positive cells can be differentiated from NKX6-1 positive pancreatic progenitor cells using the methods disclosed in Pagliuca 2014. A population of C-peptide positive/ NKX6-1 positive cells comprises at least 10%, at least 15%, at least 20%, at least 25%, at least 30%, at least 35%, at least 40%, at least 50%, at least 60%, at least 70%, at least 80% or up to about 95% of C-peptide positive/ NKX6-1 positive cells.

The cells can be harvested (e.g. spun down and resuspended in a suitable diluent) as a mixed cell culture.

In certain embodiments, the in vitro method further comprises enriching and/or isolating for example the PDX1/NKX6-1 double positive pancreatic progenitor cells and/or C-Peptide/NKX6-1 double positive pancreatic beta cells.

In an embodiment, the isolating step comprises contacting the population of cells with a specific agent that binds NKX6-1 positive cells.

It has also been demonstrated for example, in Example 4, that antibodies directed to GP2, CD142, HPx1 and HPx2 can be used to enrich for NKX6-1 positive cells.

Accordingly in an embodiment, the in vitro method comprises contacting a population of cells comprising NKX6-1 positive cells with an HPX epitope detecting antibody.

### In vitro use of Compositions

The components for differentiating PDX1/NKX6-1 double positive pancreatic progenitor cells from PDX1+ endodermal cells can be comprised as a single supplement to be added to base media such DMEM (or for example IMDM, RPMI, CMRL). The supplement would contain in an embodiment the active ingredients: BMP inhibitor, EGF, a tankyrase inhibitor that selectively binds to the adenosine subsite of tankyrase enzymes, Ascorbic Acid, L-Glutamine and B27.

A further aspect is the in vitro use of a culture medium composition suitable for the production of PDX1+/NKX6-1+ pancreatic progenitor cells from a PDX1+ endodermal cell population, as further defined in the claims. A suitable culture medium composition comprises EGF, and a tankyrase inhibitor that selectively binds to the adenosine subsite of a tankyrase enzyme, optionally a BMP inhibitor component, and/or additionally other growth factors etc. described herein.

The term "cell culture medium" (also referred to herein as a "culture medium" or "medium") as referred to herein is a medium for culturing cells containing nutrients that maintain cell viability and support proliferation and optionally differentiation. The cell culture medium may contain any of the following in an appropriate combination: salt(s), buffer(s), amino acids, glucose or other sugar(s), antibiotics, serum or serum replacement, and other components such as peptide growth factors, vitamins etc. Cell culture media ordinarily used for particular cell types are known to those skilled in the art.

The suitable culture medium can include a suitable base culture medium including for example DMEM (Life Technologies), IMDM, RPMI, CMRL and/or any other or media that supports the growth of endodermal cells to provide for example a base culture medium composition to which components and optionally other agents can be added (e.g. to provide a suitable Stage 4 culture medium composition).

The culture medium is used to culture a population of PDX1+ endoderm cells to induce PDX1/NKX6-1 double positive differentiation.

In some embodiments, the medium composition (or base medium) can further comprise one or more antibiotics such as penicillin and streptomycin; one or more amino acids such as L-Glutamine (Life Technologies), the serum substitute B27 Supplement (Life Technologies) and/or one or more vitamins such as L-ascorbic acid (SIGMA).

The amount of the components in the supplement can for example be amounts that when diluted in a culture medium (e.g. when diluted in a 450 mL base medium) result in concentrations described herein for differentiating for example an endodermal population. Similarly, the concentration of the components in the culture medium can be concentrations described herein for differentiating for example an endodermal population.

### Uses

The PDX1+/NKX6-1+ HPSC-derived pancreatic progenitors and their beta cells derivatives could be used for a number of applications.

For example, the PDX1+/NKX6-1 pancreatic progenitor cells can be used for predictive drug toxicology and drug discovery.

Accordingly, the cells can be used in an assay comprising: contacting PDX1+/NKX6-1-expressing population generated using a method described herein with a test compound, and determining if the test compound: 1) stimulates expansion, 2) triggers apoptosis, and/or 3) induces differentiation of the PDX1/NKX6-1 expressing cells to insulin producing beta-like cells to other hormone-producing cells (e.g. somatostatin, glucagon, pancreatic polypeptide and ghrelin), acinar or ductal cells; compared to a control. Assays known in the art can be used for example to assess apoptosis and/or cell expansion. Differentiation to hormone producing cells can be assessed by measuring hormone production, optionally secreted hormone or by assessing mRNA levels.

The use of an HPX antibody can facilitate the compound screening, by providing a more homogenous population for contacting with the test compound.

The cells described can also be used for cell transplantation. For example, mixed population of cells, enriched and/or isolated PDX1/NKX6-1 double positive pancreatic progenitor cells and/or NKX6-1 positive insulin producing cells can be introduced into a subject in need thereof, for example for treating diabetes and/or a pre-diabetes condition.

Accordingly, cells can be obtained and/or pancreatic precursor cells and/or insulin producing cells can be prepared according to a method described herein, and said cells can be used for administration to a subject in need thereof, for example a subject with diabetes.

Said cells and compositions comprising said cells can be used for transplanting and/or treating a subject in need thereof, for example can be used for transplanting and/or treating a subject with diabetes, such as type I diabetes or type II diabetes.

The PDX1/NKX6-1 double positive pancreatic progenitor cells and/or beta cells can be used in tissue engineering. For example, access to purified populations of pancreatic lineage cells will enable the generation of engineered constructs with defined numbers of pancreatic cells.

The methods can be applied to patient specific disease hiPSCs and used for example to model diabetes. For example, pancreatic or other cells from a patient with diabetes can be isolated, treated to obtain hiPSCs which can then be cultured and induced to differentiate to PDX1/NKX6-1 double positive pancreatic progenitor cells. These cells can be used to assess characteristics of the disease, such as the genes involved in the disease or the response to patients' immune cells.

For example, normal cells and patient specific disease hiPSCs can be induced to PDX1/NKX6-1 double positive pancreatic cells and compared to other cell types. For example, genetic, epigenetic and proteomic analyses of pancreatic progenitors and beta cells from normal and patient specific hiPSCs can be conducted. Such detailed analyses can lead to the discovery of signaling pathways, transcriptional regulatory networks and/or cell surface markers that regulate normal human pancreatic development as well as those that play a role in disease.

The term "subject" or "patient" as used herein includes all members of the animal kingdom including mammals, and suitably refers to humans.

The terms "treat", "treating", "treatment", etc., as applied to an *in vitro* cell culture, include subjecting the culture medium to any kind of process or condition, or addition or removal of any compounds to the culture medium. As applied to an isolated cell, include subjecting the cell to any kind of process or condition or performing any kind of manipulation or procedure on the cell. As applied to a subject, the terms refer to providing medical or surgical attention, care, or management to an individual.

The term "treatment" as used herein as applied to a subject, refers to an approach aimed at obtaining beneficial or desired results, including clinical results and includes medical procedures and applications including for example pharmaceutical interventions, surgery, radiotherapy and naturopathic interventions as well as test treatments for treating diabetes. Beneficial or desired clinical results can include, but are not limited to, alleviation or amelioration of one or more symptoms or conditions, diminishment of extent of disease, stabilized (i.e. not worsening) state of disease, preventing spread of disease, delay or slowing of disease progression, remission from a disease state, amelioration or palliation of the disease state, and remission (whether partial or total), whether detectable or undetectable. "Treatment" can also mean prolonging survival as compared to expected survival if not receiving treatment.

As used herein, the terms "administering," "introducing" and "transplanting" are used interchangeably in the context of delivering cells (e.g. PDX1/NKX6-1 double positive pancreatic progenitor cells, or their differentiated progeny (e.g. insulin-producing cells such as pancreatic .beta.-cells) into a subject, by a method or route which results in at least partial localization of the introduced cells at a desired site. The cells can be implanted directly to the pancreas, or alternatively be administered by any appropriate route which results in delivery to a desired location in the subject where at least a portion of the implanted cells or components of the cells remain viable.

Further, the definitions and embodiments described in particular sections are intended to be applicable to other embodiments herein described for which they are suitable as would be understood by a person skilled in the art. For example, in the following passages, different aspects of the invention are defined in more detail. Each aspect so defined may be combined with any other aspect or aspects unless clearly indicated to the contrary. In particular, any feature indicated as being preferred or advantageous may be combined with any other feature or features indicated as being preferred or advantageous.

The above disclosure generally describes the present application. A more complete understanding can be obtained by reference to the following specific examples. These examples are described solely for the purpose of illustration and are not intended to limit the scope of the claims. Changes in form and substitution of equivalents are contemplated as circumstances might suggest or render expedient. Although specific terms have been employed herein, such terms are intended in a descriptive sense and not for purposes of limitation.

The following non-limiting examples are illustrative of the present disclosure. The references to methods of treatment by therapy or surgery in Examples 3 to 5 of this description are to be interpreted as reference to compounds, pharmaceutical compositions and medicaments obtainable by the present invention for use in those methods.

### Examples

### Example 1

### Tankyrase inhibitors induce PDX1+/NKX6-1+ pancreatic progenitor cells that differentiates into insulin producing cells

In this report we present a new in vitro method to generate PDX1+/NKX6.1+ endoderm from a variety of hPSC lines. Using a GFP reporter line for the INS locus, we have demonstrated that despite tankyrase inhibitors generated similar pancreatic progenitor populations, cells treated with selective tankyrase inhibitors such as IWR-1 and WIKI4 could generate higher percentages of Insulin:GFP+ cells on day 21 of differentiation. (See Figures 1-5).

### Example 2 [Reference example]

### Specific ARDT inhibitors that target ARDT 5 and 6 (TANK1 and TANK2) give rise to pancreatic progenitor cells that generate beta-like cells using H1 cell line.

The tested ARDT inhibitors antagonize tankyrase signaling by targeting the adenosine-subsite of tankyrase proteins (also known as ARDT5/6). Conversely, cells treated with a compound, MK4827, that only inhibits PARP1/2 generated a population similar to DMSO control [Figure 5]. By culturing these tankyrease inhibitors-derived pancreatic progenitors using published protocol (Rezania 2014, Paglica 2014), we could generate c-peptide+/NKX6.1+ beta-like populations on day 23. (See Figures 6 and 7).

### Example 3

### WIKI4-generated pancreatic progenitors developed into endocrine, exocrine and ductal cells in vivo

In order to evaluate their developmental potential in vivo, the WIKI4-generated pancreatic progenitors were transplanted subcutaneously in immunocompromised mice. Grafts were analyzed 3 months post-transplantation and stained for insulin, NKX6-1, Glucagon, Cytokeratin 19 and trypsin. Immunofluorescence staining demonstrated that WIKI4-treated cells have the potential to develop into beta-like cells (Insulin+/NKX6-1+), alpha-like cells (Glucagon+/lnsulin-), trypsin+ and cytokeratin19+ cells, demonstrating the presence of both endocrine, acinar and ductal cells (see Figure 8).

### Example 4

### WIK14-generated beta-like cells developed into endocrine cells in vivo

In order to evaluate their developmental potential in vivo, the WIKI4-generated beta-like cell-containing cultures were transplanted subcutaneously or under the kidney capsule in immunocompromised mice. Grafts were analyzed 7.5 weeks post-transplantation and stained for insulin, Glucagon, and somatostatin. Immunofluorescence staining demonstrated that WIKI4-treated cells have the potential to develop into islet-like cells characterized by beta-like cells (Insulin+), alpha-like cells (Glucagon+) and delta-like cells (somatostatin+) (see Figure 9).

### Example 5

### WIK14-generated beta-like cells normalize glycaemia in animal model of diabetes

In order to evaluate their functionality in vivo, the WIKI4-generated beta-like cell-containing cultures were transplanted subcutaneously or under the kidney capsule in immunocompromised mice rendered hyperglyceamic with streptozotocin injection. Blood glucose was normalized initially with insulin pellets (weeks-0-4 and 8-12, kidney capsule and weeks 0-4 and 10-14, subcutaneous) and subsequently by the transplanted cells, demonstrating their ability to sense glucose and release insulin accordingly (see Figure 10).

While the present application has been described with reference to what are presently considered to be the preferred examples, it is to be understood that the application is not limited to the disclosed examples. To the contrary, the application is intended to cover various modifications and equivalent arrangements included within the scope of the appended claims.

### REFERENCE LIST

1. Pagliuca FW, Millman JR, Gürtler M, Segel M, Van Dervort A, Ryu JH, Peterson QP, Greiner D, Melton DA. Generation of functional human pancreatic β cells in vitro. Cell. 2014 Oct 9;159(2):428-39. doi: 10.1016/j.cell.2014.09.040.
2. Bitterman, K.J., Anderson, R.M., Cohen, H.Y., Latorre-Esteves, M. and Sinclair, D.A. 2002 Inhibition of Silencing and Accelerated Aging by Nicotinamide, a putative negative regulator of yest Sir2 and Human SIRT1. JBC 277(47): 45099
3. Dorrell, C., Abraham, S.L., Lanxon-Cookson, K.M., Canaday, P.S., Streeter, P.R., and Grompe, M. 2008. Isolation of major pancreatic cell types and long-term culture-initiating cells using novel human surface markers. Stem Cell Res 1(3): 183-194.
4. Jaramillo, M. and Banerjee, I. 2012. Endothelial Cell Co-culture Mediates Maturation of Human Embryonic Stem Cell to Pancreatic Insulin Producing Cells in a Directed Differentiation Approach. J Vis Exp(61).
5. Kelly, O.G., Chan, M.Y., Martinson, L.A., Kadoya, K., Ostertag, T.M., Ross, K.G., Richardson, M., Carpenter, M.K., D'Amour, K.A., Kroon, E., Moorman, M., Baetge, E.E., and Bang, A.G. 2011. Cell-surface markers for the isolation of pancreatic cell types derived from human embryonic stem cells. Nat Biotechnol 29(8): 750-756.
6. Lammert, E., Cleaver, O., and Melton, D. 2001. Induction of pancreatic differentiation by signals from blood vessels. Science 294(5542): 564-567.
7. Nostro, M.C., Sarangi, F., Ogawa, S., Holtzinger, A., Corneo, B., Li, X., Micallef, S.J., Park, I.H., Basford, C., Wheeler, M.B., Daley, G.Q., Elefanty, A.G., Stanley, E.G., and Keller, G. 2011. Stage-specific signaling through TGFbeta family members and WNT regulates patterning and pancreatic specification of human pluripotent stem cells. Development 138(5): 861-871.
8. Otonkoski, T., Beattie, G.M., Mally, M.I., Ricordi, C., and Hayek, A. 1993. Nicotinamide is a potent inducer of endocrine differentiation in cultured human fetal pancreatic cells. J Clin Invest 92(3): 1459-1466.
9. Sander, M., Sussel, L., Conners, J., Scheel, D., Kalamaras, J., Dela Cruz, F., Schwitzgebel, V., Hayes-Jordan, A., and German, M. 2000. Homeobox gene NKX6-1 lies downstream of Nkx2.2 in the major pathway of beta-cell formation in the pancreas. Development 127(24): 5533-5540.
10. Yoshitomi, H. and Zaret, K.S. 2004. Endothelial cell interactions initiate dorsal pancreas development by selectively inducing the transcription factor Ptf1a. Development 131(4): 807-817.
11. Huang, S. M. A., Mishina, Y. M., Liu, S., Cheung, A., Stegmeier, F., Michaud, G. A., ... & Cong, F. (2009). Tankyrase inhibition stabilizes axin and antagonizes Wnt signalling. Nature, 461(7264), 614-620.
12. Lehtiö, L., Chi, N. W., & Krauss, S. (2013). Tankyrases as drug targets. Febs Journal, 280(15), 3576-3593.
13. Riffel;, J.L., Lord, C.J., & Ashworth, A. (2012). Tankyrase-targeted therapeutics: expanding opportunities in the PARP family. Nature Reviews, 11, 923-936.
14. Micallef, S. J., Li, X., Schiesser, J. V., Hirst, C. E., Yu, Q. C., Lim, S. M., ... & Stanley, E. G. (2012). INS GFP/w human embryonic stem cells facilitate isolation of in vitro derived insulin-producing cells. Diabetologia, 55(3), 694-706.
15. Nostro, M. C., Sarangi, F., Yang, C., Holland, A., Elefanty, A. G., Stanley, E. G., ... & Keller, G. (2015). Efficient Generation of NKX6-1+ Pancreatic Progenitors from Multiple Human Pluripotent Stem Cell Lines. Stem cell reports, 4(4), 591-604.
16. Rezania, A., Bruin, J. E., Arora, P., Rubin, A., Batushansky, I., Asadi, A., ... & Kieffer, T. J. (2014). Reversal of diabetes with insulin-producing cells derived in vitro from human pluripotent stem cells. Nature biotechnology*.*
17. Anumala, U. R., Waaler, J., Nkizinkiko, Y., Ignatev, A., Lazarow, K., Lindemann, P., Olsen, P. A., Murthy, S., Obaji, E., Majouga, A. G., Leonov, S., von Kries, J. P., Lehtiö, L., Krauss, S., and Nazaré, M. Discovery of a Novel Series of Tankyrase Inhibitors by a Hybridization Approach. J. Med. Chem. 2017, 60, 10013-10025.
18. Ameri J, Borup R, Prawiro C, Ramond C, Schachter KA, Scharfmann R, Semb H. Efficient generation of glucose-responsive beta cells from isolated GP2+ human pancreatic progenitors. Cell Rep. 2017 Apr 4;19(1):36-49. doi: 10.1016/j.celrep.2017.03.032.
19. Ramond C, Glaser N, Berthault C, Ameri J, Kirkegaard JS, Hansson M, Honoré C, Semb H, Scharfmann R. Reconstructing human pancreatic differentiation by mapping specific cell populations during development. Elife. 2017 Jul 21;6. pii: e27564. doi: 10.7554/eLife.27564.
20. Cogger KF, Sinha A, Sarangi F, McGaugh EC, Saunders D, Dorrell C, Mejia-Guerrero S, Aghazadeh Y, Rourke JL, Screaton RA, Grompe M, Streeter PR, Powers AC, Brissova M, Kislinger T, Nostro MC. Glycoprotein 2 is a specific cell surface marker of human pancreatic progenitors. Nat Commun. 2017 Aug 24;8(1):331. doi: 10.1038/s41467-017-00561-0.

## Claims

1. An in vitro method of producing PDX1+/NKX6-1+ pancreatic progenitor cells from a PDX1+ endodermal cell population, the method comprising contacting the endodermal cell population with:
an agent that activates ErbB1; and
a tankyrase inhibitor that selectively binds to the adenosine subsite of a tankyrase enzyme and has a higher affinity to the adenosine subsite than a nicotinamide subsite of the tankyrase enzyme, wherein the tankyrase enzyme is TNK1 or TNK2 and the tankyrase inhibitor is WIKI4, G007-LK, JW74, JW55, CMP24, CMP40, or CMP4, or a salt, solvate and/or conjugate thereof;
to induce the differentiation of at least a portion of the PDX1+ endodermal cell population into PDX1+NKX6-1+ pancreatic progenitor cells.

2. The in vitro method of claim 1, wherein the tankyrase inhibitor does not bind to a nicotinamide subsite of the tankyrase enzyme.

3. The in vitro method of claim 1, further comprising contacting the PDX1+ endodermal cell population with a BMP inhibitor component.

4. The in vitro method of claim 3, wherein the BMP inhibitor component is Noggin, Dorsomorphin, LDN, CHORDIN, BMPR1A, or BMPR1B.

5. The in vitro method of any one of claim 1-4, wherein the tankyrase inhibitor is WIKI4, G007-LK, JW74, or JW55, or a salt, solvate and/or conjugate thereof.

6. The in vitro method of claim 5, comprising contacting the PDX1+ endodermal cell population with Noggin, EGF and one of WIKI4, G007-LK, JW74 and JW55 or contacting the PDX1+ endodermal cell population with Noggin, EGF and at least one of G007-LK, JW74 or JW55 and WIKI4.

7. The in vitro method of any one of claims 1 to 6, wherein the endodermal PDX1+ cell population is differentiated from pluripotent stem cells (PSCs) such as an embryonic stem cell (ESC) or an induced pluripotent stem cells (iPSCs), preferably wherein the pluripotent stem cell is a human ESC (hESC) or a human iPSC (hiPSC).

8. The in vitro method of any one of claims 1 to 7, first comprising producing the PDX1+ endodermal cell population, preferably wherein producing the PDX1+ endodermal cell population comprises one or more of steps:
a. contacting a pluripotent stem cell population with a combination of:
I. a nodal agonist, optionally ActA and a wnt signaling agonist, optionally, Wnt3a or CIHR 99021,
II. a nodal agonist, optionally Act A, a FGF agonist, optionally bFGF and optionally a wnt signaling agonist, optionally Wnt3a CIHR 99021,; and
III. a nodal agonist, optionally ActA and a FGF agonist, optionally bFGF;
to produce Stage 1 differentiated cells;
b. contacting the Stage 1 differentiated cells with a FGF agonist, optionally FGF10, and optionally wnt signaling agonist, optionally Wnt3a and/or noggin to produce Stage 2 differentiated cells; and
c. contacting the Stage 2 differentiated cells with Noggin and optionally cyclopamine-KAAD (Cyc), a FGF agonist, optionally FGF10 and/or an Exendin-4 component, optionally Exendin-4 to provide a endodermal cell population.

9. The in vitro method of any one of claims 1-8, further comprising differentiating the PDX1+/NKX6-1+ pancreatic progenitor cells into an insulin producing cell population, preferably wherein the insulin producing cell population comprises at least 25%, at least 30%, at least 35%, at least 40%, at least 50%, at least 60%, at least 70%, at least 80% or up to about 95% insulin producing cells or the insulin producing cell population comprises at least 25%, at least 30%, at least 35%, at least 40%, at least 50%, at least 60%, at least 70%, at least 80% or up to about 95% C-Peptide+/NKX6.1+ cells.

10. The in vitro method of any one of claims 1 to 9, further comprising enriching and/or isolating a PDX1+/NKX6-1+ progenitor cell population before differentiation.

11. In vitro use of a culture composition for the production of PDX1+/NKX6-1+ pancreatic progenitor cells from a PDX1+ endodermal cell population, the composition comprising a suitable base culture medium, EGF, a tankyrase inhibitor that selectively binds to the adenosine subsite of a tankyrase enzyme, and optionally a BMP inhibitor.

## Patentansprüche

1. In-vitro-Verfahren zur Herstellung von PDX1+/NKX6-1+-Pankreas-Vorläuferzellen von einer PDX1+-Endodermzellenpopulation, wobei das Verfahren das Kontaktieren der Endodermzellenpopulation mit Folgendem umfasst:
einem Wirkstoff, der ErbB1 aktiviert; und
einem Tankyrase-Inhibitor, der sich selektiv an die Adenosin-Bindungsstelle eines Tankyrase-Enzyms bindet und eine höhere Affinität gegenüber der Adenosin-Bindungsstelle aufweist als eine Nicotinamid-Bindungsstelle des Tankyrase-Enzyms, wobei das Tankyrase-Enzym TNK1 oder TNK2 ist und der Tankyrase-Inhibitor WIKI4, G007-LK, JW74, JW55, CMP24, CMP40 oder CMP4 oder ein Salz, Solvat und/oder Konjugat davon ist;
zum Induzieren der Differenzierung mindestens eines Teils der PDX1+-Endodermzellenpopulation in PDX1+/NKX6-1+-Pankreas-Vorläuferzellen.

2. In-vitro-Verfahren nach Anspruch 1, wobei der Tankyrase-Inhibitor sich nicht an eine Nicotinamid-Bindungsstelle des Tankyrase-Enzyms bindet.

3. In-vitro-Verfahren nach Anspruch 1, ferner umfassend das Kontaktieren der PDX1+-Endodermzellenpopulation mit einer BMP-Inhibitor-Komponente.

4. In-vitro-Verfahren nach Anspruch 3, wobei der BMP-Inhibitor Noggin, Dorsomorphin, LDN, CHORDIN, BMPR1A oder BMPR1B ist.

5. In-vitro-Verfahren nach einem der Ansprüche 1 bis 4, wobei der Tankyrase-Inhibitor WIKI4, G007-LK, JW74 oder JW55 oder ein Salz, Solvat und/oder Konjugat davon ist.

6. In-vitro-Verfahren nach Anspruch 5, umfassend das Kontaktieren der PDX1+-Endodermzellenpopulation mit Noggin, EGF und einem von WIKI4, G007-LK, JW74 und JW55 oder das Kontaktieren der PDX1+-Endodermzellenpopulation mit Noggin, EGF und mindestens einem von G007-LK, JW74, JW55 und WIKI4.

7. In-vitro-Verfahren nach einem der Ansprüche 1 bis 6, wobei die PDX1+-Endodermzellenpopulation von pluripotenten Stammzellen (PSCs), wie beispielsweise einer embryonalen Stammzelle (ESC) oder einer induzierten pluripotenten Stammzelle (iPSCs), differenziert wird, wobei die pluripotente Stammzelle vorzugsweise eine humane ESC (hESC) oder eine humane iPSC (hiPSC) ist.

8. In-vitro-Verfahren nach einem der Ansprüche 1 bis 7, das zuerst die Herstellung der PDX1+-Endodermzellenpopulation umfasst, wobei die Herstellung der PDX1+-Endodermzellenpopulation vorzugsweise einen oder mehrere der folgenden Schritte umfasst:
a. Kontaktieren einer pluripotenten Stammzellenpopulation mit einer Kombination aus:
I. einem Nodal-Agonisten, wahlweise ActA, und einem wnt-Signal-Agonisten, wahlweise Wnt3a oder CIHR 99021,
II. einem Nodal-Agonisten, wahlweise Act A, einem FGF-Agonisten, wahlweise bFGF, und wahlweise einem wnt-Signal-Agonisten, wahlweise Wnt3a CIHR 99021; und
III. einem Nodal-Agonisten, wahlweise ActA, und einem FGF-Agonisten, wahlweise bFGF;
zur Herstellung von Zellen im Stadium 1 der Differenzierung;
b. Kontaktieren der Zellen im Stadium 1 der Differenzierung mit einem FGF-Agonisten, wahlweise FGF10, und wahlweise wnt-Signal-Agonisten, wahlweise Wnt3a und/oder Noggin, zur Herstellung von Zellen im Stadium 2 der Differenzierung; und
c. Kontaktieren der Zellen im Stadium 2 der Differenzierung mit Noggin und wahlweise Cyclopamin-KAAD (Cyc), einem FGF-Agonisten, wahlweise FGF10, und/oder einer Exendin-4-Komponente, wahlweise Exendin-4, zum Bereitstellen einer Endodermzellenpopulation.

9. In-vitro-Verfahren nach einem der Ansprüche 1 bis 8, ferner umfassend das Differenzieren der PDX1+/NKX6-1+-Pankreas-Vorläuferzellen in eine insulinproduzierende Zellenpopulation, wobei die insulinproduzierende Zellenpopulation vorzugsweise mindestens 25 %, mindestens 30 %, mindestens 35 %, mindestens 40 %, mindestens 50 %, mindestens 60 %, mindestens 70 %, mindestens 80 % oder bis zu etwa 95 % insulinproduzierende Zellen umfasst oder die insulinproduzierende Zellenpopulation mindestens 25 %, mindestens 30 %, mindestens 35 %, mindestens 40 %, mindestens 50 %, mindestens 60 %, mindestens 70 %, mindestens 80 % oder bis zu etwa 95 % C-Peptid+/NKX6.1+-Zellen umfasst.

10. In-vitro-Verfahren nach einem der Ansprüche 1 bis 9, das ferner das Anreichern und/oder Isolieren einer PDX1+/NKX6-1+-Vorläufer-Zellenpopulation vor der Differenzierung umfasst.

11. In-vitro-Verwendung einer Kulturzusammensetzung zur Herstellung von PDX1+/NKX6-1+-Pankreas-Vorläuferzellen von einer PDX1+-Endodermzellenpopulation, wobei die Zusammensetzung ein geeignetes Basiskulturmedium, EGF, einen Tankyrase-Inhibitor, der sich selektiv an die Adenosin-Bindungsstelle eines Tankyrase-Enzyms bindet, und wahlweise einen BMP-Inhibitor umfasst.

## Revendications

1. Procédé in vitro de production de cellules progénitrices pancréatiques PDX1+/NKX6-1+ à partir d'une population de cellules endodermiques PDX1+, le procédé comprenant la mise en contact de la population de cellules endodermiques avec :
un agent qui active ErbB1 ; et
un inhibiteur de tankyrase qui se lie sélectivement au sous-site de l'adénosine d'une enzyme tankyrase et a une affinité pour le sous-site de l'adénosine supérieure à celle pour un sous-site du nicotinamide de l'enzyme tankyrase, dans lequel l'enzyme tankyrase est TNK1 ou TNK2 et l'inhibiteur de tankyrase est WIKI4, G007-LK, JW74, JW55, CMP24, CMP40 ou CMP4, ou un sel, solvate et/ou conjugué de celui-ci ;
pour induire la différenciation d'au moins une portion de la population de cellules endodermiques PDX1+ en cellules progénitrices pancréatiques PDX1+NKX6-1+.

2. Procédé in vitro selon la revendication 1, dans lequel l'inhibiteur de tankyrase ne se lie pas à un sous-site du nicotinamide de l'enzyme tankyrase.

3. Procédé in vitro selon la revendication 1, comprenant en outre la mise en contact de la population de cellules endodermiques PDX1+ avec un composant inhibiteur de BMP.

4. Procédé in vitro selon la revendication 3, dans lequel le composant inhibiteur de BMP est la noggine, la dorsomorphine, LDN, CHORDIN, BMPR1A ou BMPR1B.

5. Procédé in vitro selon l'une quelconque des revendications 1 à 4, dans lequel l'inhibiteur de tankyrase est WIKI4, G007-LK, JW74 ou JW55, ou un sel, solvate et/ou conjugué de celui-ci.

6. Procédé in vitro selon la revendication 5, comprenant la mise en contact de la population de cellules endodermiques PDX1+ avec de la noggine, de l'EGF et l'un parmi WIKI4, G007-LK, JW74 et JW55 ou la mise en contact de la population de cellules endodermiques PDX1+ avec de la noggine, de l'EGF et au moins l'un parmi G007-LK, JW74 ou JW55 et WIKI4.

7. Procédé in vitro selon l'une quelconque des revendications 1 à 6, dans lequel la population de cellules endodermiques PDX1+ est différenciée à partir de cellules souches pluripotentes (PSC) telles qu'une cellule souche embryonnaire (ESC) ou une cellule souche pluripotente induite (iPSC), de préférence dans lequel la cellule souche pluripotente est une ESC humaine (hESC) ou une iPSC humaine (hiPSC).

8. Procédé in vitro selon l'une quelconque des revendications 1 à 7, comprenant en premier lieu la production de la population de cellules endodermiques PDX1+, de préférence dans lequel la production de la population de cellules endodermiques PDX1+ comprend une ou plusieurs des étapes suivantes :
a. mise en contact d'une population de cellules souches pluripotentes avec une combinaison de :
I. un agoniste nodal, éventuellement ActA et un agoniste de signalisation de wnt, éventuellement Wnt3a ou CIHR 99021,
II. un agoniste nodal, éventuellement Act A, un agoniste de FGF, éventuellement bFGF et éventuellement un agoniste de signalisation de wnt, éventuellement Wnt3a ou CIHR 99021, et
III. un agoniste nodal, éventuellement ActA et un agoniste de FGF, éventuellement bFGF ;
pour produire des cellules différenciées de stade 1 ;
b. mise en contact des cellules différenciées de stade 1 avec un agoniste de FGF, éventuellement FGF10, et éventuellement un agoniste de signalisation de wnt, éventuellement Wnt3a et/ou de la noggine pour produire des cellules différenciées de stade 2 ; et
c. mise en contact des cellules différenciées de stade 2 avec de la noggine et éventuellement de la cyclopamine-KAAD (Cyc), un agoniste de FGF, éventuellement FGF10 et/ou un composant d'exendine-4, éventuellement l'exendine-4, pour former une population de cellules endodermiques.

9. Procédé in vitro selon l'une quelconque des revendications 1 à 8, comprenant en outre la différenciation des cellules progénitrices pancréatiques PDX1+/NKX6-1+ en une population de cellules productrices d'insuline, de préférence dans lequel la population de cellules productrices d'insuline comprend au moins 25 %, au moins 30 %, au moins 35 %, au moins 40 %, au moins 50 %, au moins 60 %, au moins 70 %, au moins 80 % ou jusqu'à environ 95 % de cellules productrices d'insuline, ou la population de cellules productrices d'insuline comprend au moins 25 %, au moins 30 %, au moins 35 %, au moins 40 %, au moins 50 %, au moins 60 %, au moins 70 %, au moins 80 % ou jusqu'à environ 95 % de cellules C-Peptide+/NKX6.1+.

10. Procédé in vitro selon l'une quelconque des revendications 1 à 9, comprenant en outre l'enrichissement en et/ou l'isolation d'une population de cellules progénitrices pancréatiques PDX1+/NKX6-1+ avant différenciation.

11. Utilisation in vitro d'une composition de culture pour la production de cellules progénitrices pancréatiques PDX1+/NKX6-1+ à partir d'une population de cellules endodermiques PDX1+, la composition comprenant un milieu de culture de base convenable, de l'EGF, un inhibiteur de tankyrase qui se lie sélectivement au sous-site de l'adénosine d'une enzyme tankyrase, et éventuellement un inhibiteur de BMP.
